# EUROPEAN PATENT SPECIFICATION

(11) **EP 2 425 803 B1**
(45) Date of publication and mention of the grant of the patent: **03.05.2017**
(21) Application number: 10174718.6
(22) Date of filing: 31.08.2010
(51) Int. Cl.: A61F 13/15, A61F 13/56, A61F 13/64, A61F 13/66, A61F 13/74

(54) **System for forming an absorbent article**
System zur Bildung eines saugfähigen Artikels
Système pour la formation d'articles absorbants

(43) Date of publication of application: 07.03.2012
(73) Proprietor: Attends Healthcare AB, 578 24 Aneby (SE)
(72) Inventor: Gustafsson, Ida, 554 50, Jönköping (SE); Lindqvist, Arne, 152 27, Södertälje (SE); Håkansson, Mikael, 573 34, Tranås (SE); Ragnarsson, Maria, 553 20, Jönköping (SE)
(74) Representative: Awapatent AB

(56) References cited:
- EP-A2- 0 421 473
- WO-A1-2005/007052
- WO-A1-2007/071268
- WO-A1-2008/147270
- DE-A1-102004 048 540
- US-A1- 2002 193 776

## Description

### Technical Field

The present disclosure relates to a system for forming an absorbent article of pad type to be used with panties or of all-in-one type adapted to be attached around the waist of a user, and especially to a system for forming an absorbent article comprising a chassis and at least one first add-on means.

### Background

For a user of an absorbent article, such as an incontinence or menstrual protection article, it is important that the article feels safe and secure. Different users prefer or need different types of absorbent articles, such as pad types to be used with panties, or all-in-one types where wings or belts cover the waistline. Further, different users require different sizes of articles. At a care center where aiding staff helps users with different requirements using absorbent articles, a large number of articles of different types and sizes are needed. A large number of article types become very costly. Further, a large number of article types become very costly for the manufacturer of the absorbent articles.

According to WO2007/071267, an absorbent article is provided with closure means for securing the article around the waist of a user. The article is adapted to be used as a slip type article or a belted slip type article. However, the closure means for both slip and belted slip type are provided on the article when received by a user. Thereby, each absorbent article is restricted to a specified size, providing a need for a complete article type for each size requirement.

Consequently, there is a need for absorbent articles of different types and sizes to be provided and manufactured in a cost-effective way.

### Summary

It is an object of the present invention to provide an improved solution that alleviates the mentioned drawbacks with present devices to provide absorbent articles of different types and sizes in a cost-effective way. Furthermore, it is an object to provide a system for forming an absorbent article of pad type or of all-in-one type attachable around the waist of a user. The system comprises a chassis having a front portion and a back portion and a longitudinal extension along a longitudinal axis from a front end of the front portion towards a back end of the back portion, the chassis further comprising a crotch portion connecting the front portion to the back portion, the front portion having left and right front side edges, the back portion having left and right back side edges. The chassis further comprises a liquid permeable top sheet and a liquid impermeable back sheet and is enclosing an absorbent core arranged between the top sheet and the back sheet. The system further comprises at least one first add-on means adapted to be attached with a first end to the chassis such that the add-on means extend substantially transversal to the longitudinal axis of the chassis, to form an absorbent article that is attachable around the waist of a user.

The system is arranged such that the chassis is adapted to be used for forming absorbent articles both by combining a chassis with a first add-on means to form an absorbent article that is attachable around the waist of a user, and by using the chassis as a pad type absorbent article to be used with panties.

By providing a system for forming an absorbent article of different types, a more cost-effective manufacturing process may be achieved. The same chassis may be used for more than one type of absorbent article. The manufacturer may produce a large number of similar chassis, wherein some are used for pad type absorbent articles, and some for all-in-one type absorbent articles. This provides an effective manufacturing process. Further, the system may be a more cost-effective solution for a consumer that may use the same base article in a situation where an all-in-one type article is needed as in a situation where a pad type article is needed. The storage cost for a user may thereby be reduced. The chassis may have a form that allows different types of absorbent articles to be formed. Further, the first add-on means may be used together with chassis of different sizes. Thereby only one size of first add-on means may be used for all chassis sizes. The first add-on means may be adapted to have a variable length for suiting different users with different lengths around the waist. A user may have a system with a number of similar first add-on means, and a number of chassis in different sizes, providing the possibility to form absorbent articles of all-in-one type or pad type of different sizes using the same components.

By all-in-one type absorbent article is meant an absorbent article comprising all parts needed for attaching the absorbent article around the waist of a user. Such all-in-one absorbent articles may for instance be slip type articles, belt type articles, pull-on type articles or the like.

The first add-on means may be means for attaching the absorbent article around the waist of a user. The first add-on means may be wings, a belt, means for forming a pull-on type article or the like. The first add-on means may be refastenable on the chassis. The first add-on means may thereby be reusable, providing further cost-effectiveness for the user. The first add-on means may further be washable. In an alternative embodiment, the attachment of the first add-on means on the chassis may be permanent.

The top sheet and the back sheet of the chassis may be substantially larger than the absorbent core arranged between the two sheets. When using the chassis as a pad type absorbent article, the article may be used in an undergarment. The front end and the back end of the chassis may then be folded around a waist edge of the undergarment. By folding the ends of the chassis, i.e. the part of the chassis where the back sheet and the top sheet are in direct contact with each other, also called the surplus extension area of the chassis, around the waist edge of the undergarment the safety for the user is increased. Also, by having a relatively large surplus extension area that it is very easy to adjust the position of the pad such that a good fit is achieved. The large part of the chassis extending outside the extension of the absorbent core may be adapted to receive an add-on means in a secure way. By attaching the add-on means to an area outside the extension of the absorbent core, advantages may be achieved such as better attachment between the chassis and the add-on means, no disturbance of the absorption capacity in the absorbent core when an add-on means is attached, unnecessary thickness of the absorbent article at the absorbent core is avoided, and forces on the absorbent core that may risk breakage is avoided.

In one embodiment of the invention, the system may further comprise at least one second add-on means that differs from the first add-on means in size or shape, adapted to be attached to the chassis to form an absorbent article, wherein the system is arranged such that the chassis is adapted to be used for forming absorbent articles both by combining a chassis with a first add-on means to form an all-in-one type absorbent article that is attachable around the waist of a user, by combining a chassis with a second add-on means to form another type of all-in-one type absorbent article that is attachable around the waist of a user, and by using the chassis as a pad type absorbent article.

Thereby a larger flexibility of the system may be provided. The same chassis may be used for several types of absorbent articles. Thereby, the manufacturer may produce a large number of similar chassis, wherein some are used as pad type absorbent articles, and some for all-in-one absorbent articles of different types. This provides a very cost-effective solution for the manufacturer. Further, a user may choose to form different absorbent articles in all-in-one type, or pad type. This flexible solution may also be more cost-effective for the user, for instance by reducing storage costs. Further, the manufacturing process may be more cost-effective since the same chassis may be used by a user to form three different types of absorbent articles. Further, the first and second add-on means may be one-size. Thereby, the user may use the same first or second add-on means with chassis of different sizes to form all-in-one type absorbent articles. Further, the second add-on means may be wings, a belt, means for forming a pull-on type absorbent article or the like providing means for forming an all-in-one type absorbent article. The second add-on means may be of the same type of add-on means as the first add-on means, but differing in size. The second add-on means may be refastenable on the chassis. The second add-on means may thereby be reusable, providing further cost-effectiveness for the user. The second add-on means may further be washable. The attachment of the second add-on means to the chassis may alternatively be permanent.

The system may further comprise one or several additional types of add-on means that may differ from the first and the second add-on means in size or in providing additional types of absorbent articles when attached to the chassis. Such additional type of absorbent article may for instance be a pull-on type absorbent article.

In a further embodiment, the first add-on means may comprise at least one slip type wing adapted to be attached to the chassis to form an absorbent article of slip type that is attachable around the waist of a user.

A wing in an all-in-one type absorbent article may be adapted to attach the back portion of the chassis to the front portion when the absorbent article is placed around the waist of a user. The wing may be attached to the chassis to form an all-in-one type absorbent article. The first add-on means may comprise four wings adapted to be attached to the chassis. The wings may be attached at the front and back left and right side edges of the chassis. When the all-in-one type absorbent article is to be placed around the waist of a user, the four wings may be attached to each other and to the chassis to connect the front portion of the chassis to the back portion. The first add-on means may alternatively comprise two wings adapted to be attached to the left and right side edges of either the front portion or the back portion of the chassis. When attached to the side edges of the back portion, the two wings may be adapted to be attached to the front portion of the chassis when the all-in-one type absorbent article is placed around the waist of a user. The wing or wings of the first add-on means may be made of elastic material, a nonwoven material, a laminated material or combinations thereof.

In another embodiment, the second add-on means may be a belt adapted to be attached around the waist of a user and to which belt the chassis is adapted to be attached to form a belted type absorbent article that is attachable around the waist of a user.

A belt may be a belt-like strip adapted to be attached around the waist of a user. The belt may be attached to the back portion of the chassis. After the belt has been attached to the back portion of the chassis and around the waist of a user, the front portion of the chassis may be attached to the belt. As an alternative, the belt may be attached directly to the front portion of the chassis. A center part of the belt may be provided with an adhesive means, such as glue, tape, loop-hook means or the like, adapted to attach the belt to the back portion of the chassis when forming a belt type absorbent article. Alternatively, the back portion may be provided with such adhesive means adapted to receive and attach the belt to the chassis. The attachment between the belt and the back portion of the chassis when forming the belt type absorbent article may be permanent. Further, the attachment between the belt and the back portion of the chassis when forming the belt type absorbent article may be refastenable. End portions of the belt may be provided with an adhesive means, such as glue, tape, loop-hook means or the like, for attaching the belt end portions to each other around the waist of a user or to the front portion of the chassis.

A first add-on means in the form of at least one wing and a second add-on means in the form of a belt provides a cost-effective system for the manufacturer wherein the same chassis may be used to form a pad type absorbent article, a slip type absorbent article or a belt type absorbent article. The two latter when combining the chassis with a first or second add-on means respectively.

In one embodiment, the chassis may comprise at least one landing zone for receiving a first add-on means.

Such landing zones may be provided at the left and right front side edges on the chassis. Further, such landing zones may be provided at the left and right back side edges on the chassis. A landing zone may also be provided along the back end on the chassis. The at least one landing zone may further be adapted to receive a second add-on means. The landing zones on the chassis may be adapted to receive a first or second add-on means when a user forms an absorbent article of all-in-one type.

The landing zone may be provided with and/or adapted to integrate with adhesive means such as tape, glue, loop-hook means or the like. The landing zone may be napped to be adapted to attach to hook means on a first or second add-on means. Each landing zone may be adapted not to disturb the user during use of the article when no add-on means is attached to that landing zone. For instance, the landing zone may be provided with an adhesive that is not sticky unless it integrates with means on a first or second add-on means. Thereby, when the chassis is used as a pad type absorbent article, the landing zones may not affect the usage of the article. The landing zones may be arranged at parts of the chassis outside the extension area of the absorbent core. This may give the advantages of better attachment between the landing zone and the add-on means, no disturbance of the absorption capacity in the absorbent core when an add-on means is attached, unnecessary thickness of the absorbent article at the absorbent core is avoided, and forces on the absorbent core that may risk breakage is avoided. The at least one landing zone may be provided on the inside of the chassis, i.e. on the top sheet. Further, the at least one landing zone may be provided on the outside of the chassis, i.e. on the back sheet. Further, the at least one landing zone may be provided on both the inside and the outside of the chassis, i.e. both on the top sheet and the back sheet. The at least one landing zone may be provided on the entire inside or outside of the chassis, i.e. the entire top sheet or the entire back sheet.

In another embodiment, the chassis may be provided with means for attachment of the chassis to an undergarment.

Thereby, when the chassis is used as a pad type absorbent article, the means for attachment to an undergarment may facilitate the wearing of the article by keeping the article in place in the undergarment. The means for attachment of the chassis to the undergarment may be provided as glue, tape, loop-hook means or the like.

In another embodiment, the system further comprises a pad attachment means, adapted to be attached to the chassis, and adapted to facilitate the attachment of a chassis used as a pad type absorbent article to an undergarment.

The pad attachment means may be adapted to integrate with the undergarment to keep the pad type absorbent article in place in the undergarment when in use by a user. The pad type absorbent article may be a tape patch, loop-hook means, friction enhancing means or the like. The chassis may be provided with a landing zone adapted to receive the pad attachment means. This landing zone may be provided on the outside of the chassis, on the back sheet.

In a further embodiment the extension area of the chassis is in the range of about 150-300%, preferably of about 170-250% of the extension area of the absorbent core.

The extension area of the chassis is the area of a flattened chassis. The extension area of the absorbent core is the area covered by the absorbent core when the chassis is flattened out. By providing a chassis that is at least 150% of the extension area of the absorbent core, the chassis may be large enough to function not only as a pad type absorbent article, but also as a part of an all-in-one absorbent article when receiving a first or second add-on means. By having a large chassis area outside the extension area of the absorbent core, also called chassis surplus extension area, facilitates fastening add-on means, such as wings, to the chassis, for converting the chassis to an all-in one type absorbent article.

In another embodiment, there may be a distance A from a front end of the absorbent core to the front end of the chassis that is more than 5 cm, preferably 6-25 cm, more preferably 6-15 cm, and most preferably 6.5 to 9 cm.

A large chassis area outside the extension area of the absorbent core, also called chassis surplus extension area, may provide a safe and secure feeling for the user of the article. When using the chassis as a pad type absorbent article, the front end of the chassis may be folded around a waist opening of the undergarment in which the pad type article may be placed. Further, a large distance from the front end of the absorbent core to the front end of the chassis facilitates fastening add-on means, such as wings, to the chassis, for converting the chassis to an all-in one type absorbent article. On the other hand, for the absorbent product to be cost-efficient and material efficient, such a distance should not be too large.

In one embodiment, there may be a distance from a back end of the absorbent core to the back end of the chassis that is at least 5 cm, preferably 6-25 cm, more preferably 6-15 cm, and most preferably 6.5-9 cm.

A large chassis area outside the extension area of the absorbent core may provide a safe and secure feeling for the user of the article. When using the chassis as a pad type absorbent article, the back end of the chassis may be folded around a waist opening of the undergarment in which the pad type article may be placed. Further, a large distance from the back end of the absorbent core to the back end of the chassis facilitates fastening add-on means, such as wings, to the chassis, for converting the chassis to an all-in one type absorbent article.

In a further embodiment, there may be a distance from a front side edge of the absorbent core to a front side edge of the chassis that is at least 5 cm, preferably 5-25 cm, more preferably 5-15 cm, and most preferably 7-11 cm. A large distance from the front side edge of the absorbent core to the front side edge of the chassis facilitates fastening add-on means, such as wings, to the chassis, for converting the chassis to an all-in one type absorbent article. A large chassis area outside the extension area of the absorbent core may provide a safe and secure feeling for the user of the article. The area of the chassis between the side edge of the absorbent core and the front side edge of the chassis may be large enough to receive and securely hold an add-on means. Further, that area may also be provided with landing zones for receiving and securely hold an add-on means. The attachment of the add-on means on the landing zone may be refastenable. By arranging the landing zones on parts of the chassis outside the extension area of the absorbent core, some advantages may be provided. These advantages may be better attachment between the landing zone and the add-on means, no disturbance of the absorption capacity in the absorbent core when an add-on means is attached, unnecessary thickness of the absorbent article at the absorbent core is avoided, and forces on the absorbent core that may risk breakage is avoided.

In another embodiment, there may be a distance from a back side edge of the absorbent core to a back side edge of the chassis that is at least 5 cm, preferably 5-25 cm, more preferably 5-15 cm, and most preferably 5.5-9 cm.

A large chassis area outside the extension area of the absorbent core may provide a safe and secure feeling for the user of the article. The area of the chassis between the side edge of the absorbent core and the back side edge of the chassis may be large enough to receive and securely hold an add-on means. That area may also be provided with landing zones for receiving and securely hold an add-on means. The attachment of the add-on means on the landing zone may be refastenable.

### Brief Description of the Drawings

The invention will in the following be described in more detail with reference to the enclosed drawings, wherein:
Fig 1 is a schematic top view of a system according to an embodiment of the invention.
Fig 2 is cross-sectional view of an absorbing article according to the invention;
Fig 3 is a schematic top view of an absorbing article according to an embodiment of the invention;
Fig 4a is a schematic top view of an absorbent article according to an embodiment of the invention;
Fig 4b is a schematic top view of an absorbent article according to an embodiment of the invention;
Fig 5a is a schematic top view of a belt according to an embodiment of the invention;
Fig 5b is a schematic top view of an absorbent article according to an embodiment of the invention; and
Fig 6 is a schematic top view of an absorbing article according to an embodiment of the invention.

### Description of Embodiments

The present invention will be described more fully hereinafter with reference to the accompanying drawings, in which preferred embodiments of the invention are shown. This invention may, however, be embodied in many different forms and should not be construed as limited to the embodiments set forth herein; rather, these embodiments are provided so that this disclosure will be thorough and complete, and will fully convey the scope of the invention to those skilled in the art. In the drawings, like numbers refer to like elements.

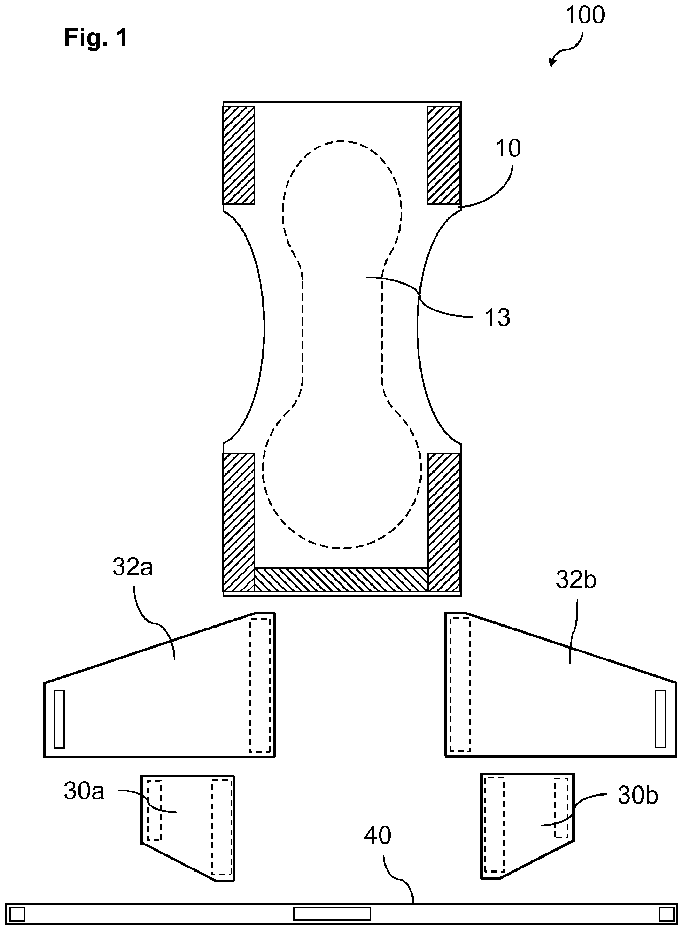

Fig 1 illustrates a system 100 according to the invention. The system 100 comprises a chassis 10 with an absorbent core 13 arranged in the chassis, between a top sheet and a back sheet of the chassis, a first add-on means and a second add-on means. The first add-on means comprises four wings 30a, 30b, 32a, 32b adapted to be attached to the chassis 10 to form an absorbent article of all-in-one type to be attached around the waist of a user. The second add-on means comprises a belt 40 adapted to be attached to the chassis 10 to form an absorbent article of belt type to be attached around the waist of a user. The chassis 10 is further adapted to be used alone as a pad type absorbent article with an undergarment.

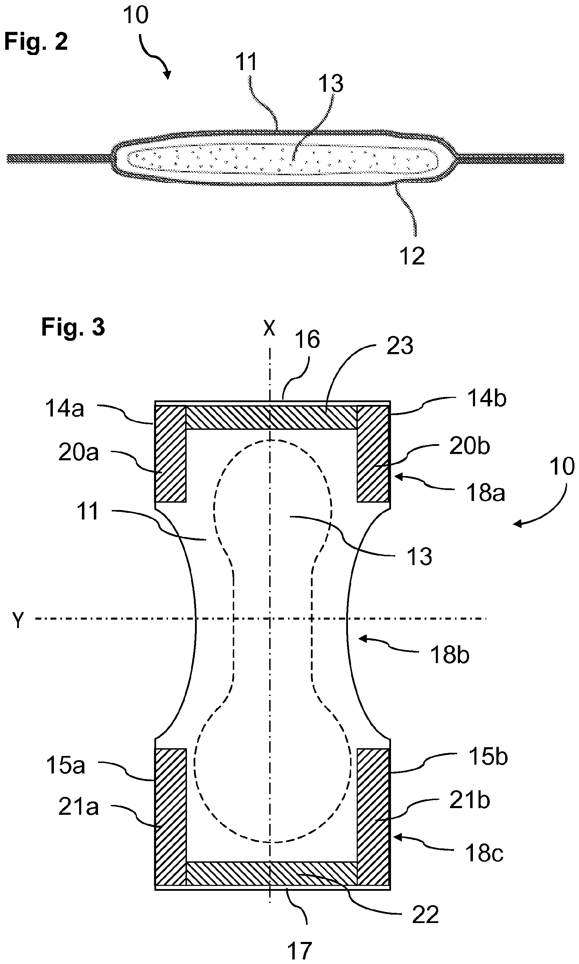

Fig 2 illustrates a cross-section at line Y in a crotch portion 18b of a chassis 10. The chassis 10 comprises a chassis consisting of a liquid permeable top sheet 11 and a liquid impermeable backsheet 12. The chassis contains an absorbent core 13. The absorbent core 13 is adapted to absorb body fluids from a user. In this example, the absorbent core 13 comprises an acquisition layer such as curly fibers, refined pulp and super absorbent material. The liquid impermeable back sheet 12 is made of plastic, breathable textile or plastic nonwoven laminate. The liquid impermeable back sheet 12 may be substantially liquid impermeable. The liquid permeable top sheet 11 is made of a nonwoven material.

Fig 3 illustrates a chassis 10 comprising a front portion 18a with a front end 16 and left and right front side edges 14a, 14b. The chassis 10 has a longitudinal extension along line X. A crotch portion 18b of the chassis 10 connects the front portion 18a with a back portion 18c. The back portion 18c comprises a back end 17 and left and right back side edges 15a, 15b. At the left front side edge 14a and at the right front side edge 14b, two left and right front landing zones 20a, 20b are provided on the top sheet 11 of the chassis 10. At the left back side edge 15a and at the right back side edge 15b, two left and right back landing zones 21 a, 21 b are provided on the top sheet 11 of the chassis 10. The landing zones 20, 21 extend substantially in parallel with the longitudinal extension direction X of the chassis 10. The left and right back landing zones 21 a, 21 b as well as the left and right front landing zones 20a, 20b may be arranged in close proximity with the closest side edge of chassis 10 or such that there is a suitable distance between the landing zone and the side edges of the chassis. At the back end 17, a back end landing zone 22 is arranged. The back end landing zone 22 extends in a direction substantially perpendicular to the longitudinal extension direction X of the chassis 10. At the front end 16 a landing zone 23 may also be arranged, in a similar way as the back end landing zone 22. The landing zones 20, 21, 22 are adapted to receive an add-on means when an absorbent article 1, 2, 3 is formed by parts from the system according to the invention. The landing zones 20, 21, 22 may be provided with adhesives such as glue, tape, loop-hook means or the like, in order to attach to an add-on means. Alternatively, the landing zones 20, 21, 22 are of a material adapted to receive and attach to such adhesives, whereby such adhesives may be arranged to an add-on means such that when the add-on means is put into contact with the landing zone the adhesives will make the add-on means attach to the landing zone.

The landing zones 20, 21, 22 are hereinafter described and illustrated as being arranged on the top sheet 11 of the chassis 10, i.e. on the inside of the chassis 10, the side facing a user during use. The landing zones 20, 21, 22 may however as well be arranged on the back sheet 12 of the chassis 10, i.e. on the outside of the chassis 10, or both on the inside and the outside of the chassis 10. Further as an alternative, some of the landing zones 20, 21, 22 may be arranged on the inside of the chassis 10 and some on the outside. In the alternative embodiments of the arrangement location of the landing zones 20, 21, 22, the add-on means in the system are arranged to be attached to the landing zones 20, 21, 22 on the inside and/or the outside of the chassis 10. As an example, the chassis 10 may be provided with landing zones both on the inside (on the top sheet 11) and on the outside (on the back sheet 12), and the add-on means may have a double patch extension in a fork-like manner, such that one patch portion may be attached to the inside landing zone and one patch portion may be attached to the outside landing zone.

The chassis 10 may further comprise not shown elastics such as leg elastics, waist elastics along the front end 16 and the back end 17, crotch elastics along the edges of the crotch portion 18b, and elastic barrier leg cuffs functioning as leakage barrier extending in parallel with the longitudinal axis X.

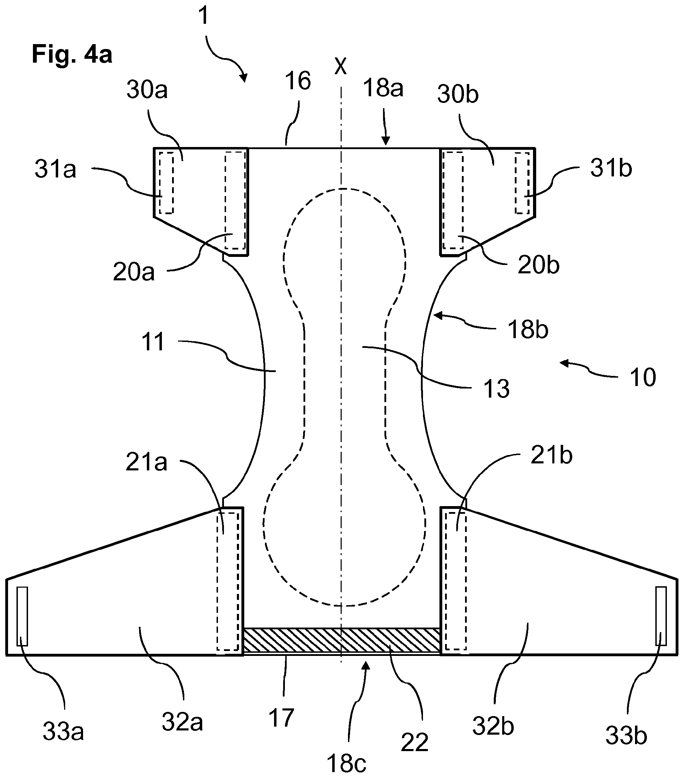

Fig 4a illustrates an absorbent article 1 formed from the system according to the invention. The absorbent article 1 is of an all-in-one type and comprises a chassis 10 and four first add-on means 30a, 30b, 32a, 32b. The chassis 10 comprises an absorbent core 13 arranged between a liquid impermeable back sheet 12 and a liquid permeable top sheet 11. The four add-on means 30, 32 comprises one left front wing 30a, one right front wing 30b, one left back wing 32a and one right back wing 32b. The front wings 30a, 30b are attached to the front portion 18a of the chassis 10 at the front landing zones 20a, 20b. The front wings 30a, 30b may be provided with means to facilitate the attachment to the landing zones 20a, 20b, said means being arranged at a location on the front wings 30a, 30b corresponding to the location of the landing zones 20a, 20b. The attachment of the front wings 30a, 30b to the chassis 10 is preferably permanent. Alternatively, the attachment of the front wings 30a, 30b to the chassis 10 may be releasable. If releasable, the wings 30a, 30b may be reusable. Further, the wings 30a, 30b may be washable. The left and right front wings 30a, 30b are preferably symmetrically arranged. In an alternative embodiment, the left front wing 30a may be larger or smaller than the right front wing 30b. To increase the fitness of the absorbent article 1 around the waist of a user, the front wings 30a, 30b could be provided with elastics. Further, to increase the flexibility in size of the front wings 30a, 30b, they could be semi-perforated such that they could be shortened when an absorbent article 1 is formed, or during attachment around the waist of a user.

The back wings 32a, 32b are attached to the chassis 10 at the back landing zones 21 a, 21 b. The back wings 32a, 32b may be provided with means to facilitate the attachment to the landing zones 21 a, 21 b, said means being arranged at a location on the back wings 32a, 32b corresponding to the location of the landing zones 21 a, 21 b. The attachment of the back wings 32a, 32b to the chassis 10 is preferably permanent. Alternatively, the attachment of the back wings 32a, 32b to the chassis 10 may be releasable. If releasable, the wings 32a, 32b may be reusable. Further, the wings 32a, 32b may be washable. The left and right back wings 32a, 32b are preferably symmetrically arranged. In an alternative embodiment, the left back wing 32a may be larger or smaller than the right back wing 32b. To increase the fitness of the absorbent article 1 around the waist of a user, the back wings 32a, 32b could be provided with elastics. Further, to increase the flexibility in size of the back wings 32a, 32b, they could be semi-perforated such that they could be shortened when an absorbent article 1 is formed, or during attachment around the waist of a user.

The front wings 30a, 30b extends a shorter distance from the chassis 10 than the back wings 32a, 32b. Alternatively, the situation may be the opposite.

The front wings 30a, 30b are provided with attachment means 31 a, 31 b at the ends of the wings facing away from the chassis 10. The attachment means 31 a, 31 b are provided on the outside of the front wings 30a, 30b, i.e. the side of the front wings 30a, 30b facing away from a user when the absorbent article 1 is attached around the waist of the user. Further, the back wings 32a, 32b are provided with attachment means 33a, 33b at the ends of the wings facing away from the chassis 10. The attachment means 33a, 33b are provided on the inside of the back wings 32a, 32b, i.e. the side of the back wings 32a, 32b facing the user when the absorbent article 1 is attached around the waist of the user. When the absorbent article 1 is being attached around the waist of a user, the front portion 18a of the chassis 10 faces the front of the user and the front wings 30a, 30b extends around the waist towards the back of the user. The back portion 18c of the chassis 10 faces the back of the user and the back wings 32a, 32b extends around the waist towards the front side of the user. The outside of the front wings 30a, 30b faces the inside of the back wings 32a, 32b. The attachment means 31 a, 31 b on the outside of the front wings 30a, 30b attaches to the inside of the back wings 32a, 32b. The back wings 32a, 32b are folded around the waist of the user such that the back wings 32a, 32b extends to the front portion of the chassis 10. The attachment means 33a, 33b on the inside of the back wings 32a, 32b are attached to the back sheet 12 of the front portion 18a of the chassis 10, adjacent to the front end 16. A waist opening is thereby formed around the user. The attachments of front wings 30a, 30b to the inside of the back wings 32a, 32b, and of the back wings 32a, 32b to the back sheet 12 of the chassis 10 are releasable, such that the absorbent article 1 is detachable from the user. The attachment means 31 a, 31 b, 33a, 33b comprises an adhesive such as glue, tape, loop-hook or the like.

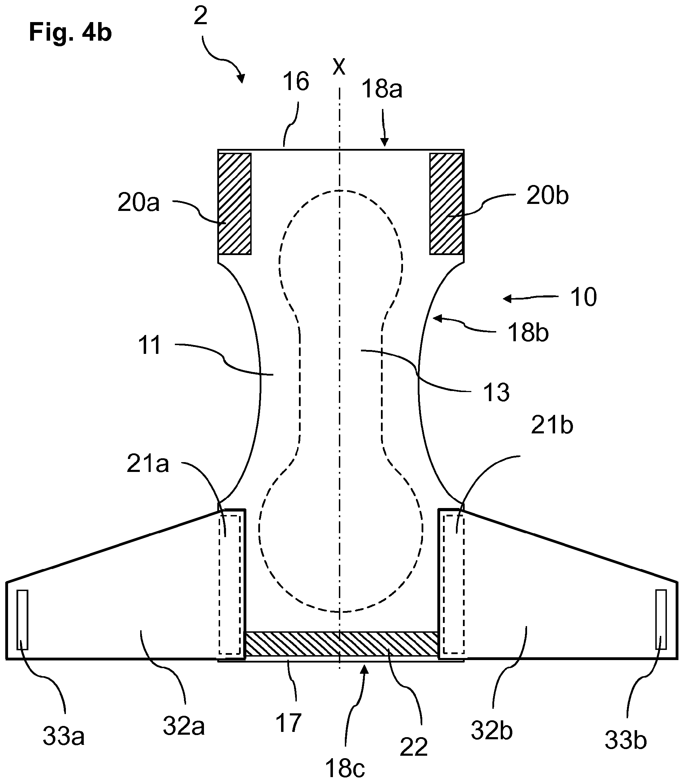

Fig 4b illustrates an alternative all-in-one type absorbent article 2 wherein only back wings 32a, 32b are attached to the chassis 10 at the landing zones 21 a, 21 b. When the absorbent article 2 is being attached around the waist of a user, the back wings 32a, 32b extends from the back of the user, around the waist to the front side to be attached to the outside of the front portion 18a of the chassis 10 by the attachment means 33a, 33b attaching to the back sheet 12. A waist opening around the user is thereby formed. The attachment of the back wings with the attachment means 33a, 33b to the back sheet 12 of the front portion 18a of the chassis 10 is releasable such that the absorbent article 2 is detachable from the user.

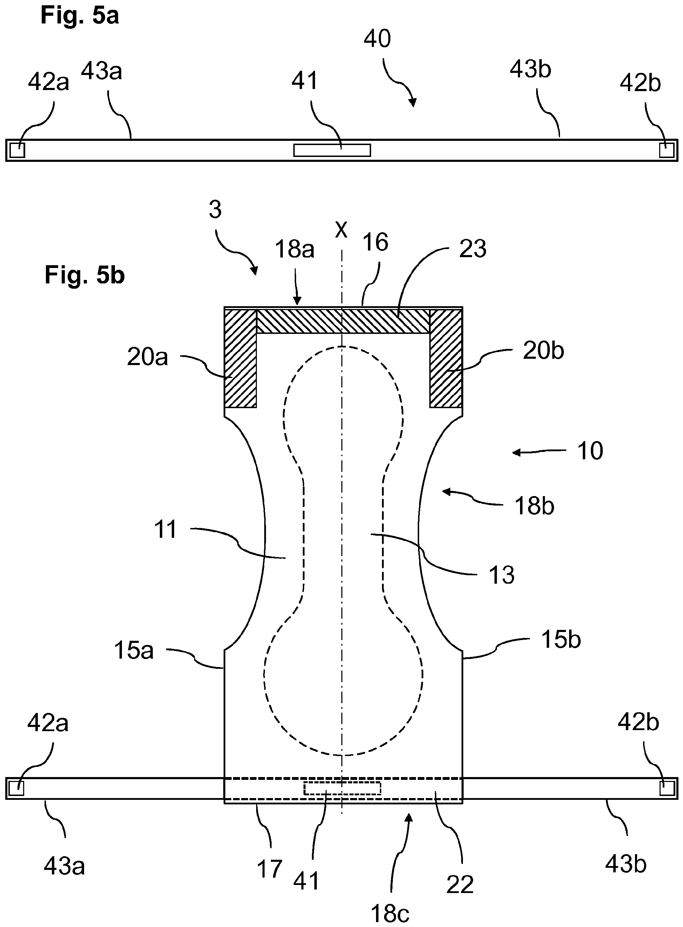

Fig 5a illustrates a belt 40 adapted to be used in the system to form an absorbent article 3 of all-in-one type, and particularly of belt type. The belt 40 is a strip comprising a center attachment means 41, a left attachment means 42a and a right attachment means 42b. The left and right attachment means 42a, 42b are provided at the ends of the left and right portions 43a, 43b of the belt 40. The center attachment means 41 is preferably loop-hook, but may in an alternative embodiment instead be glue, tape, or the like. The attachment of the center attachment means 41 is preferably releasable, for instance by using a loop-hook attachment. When using another attachment technique such as tape, glue or the like, the attachment may as well be permanent. If the attachment is releasable or permanent may be decided by the material at the landing zone on the chassis. The left and right attachment means 42a, 42b are preferably tape, but may in an alternative embodiment instead be glue, loop-hook or the like. The attachments with the left and right attachment means 42a, 42b are preferably releasable. The belt 40 is preferably of a nonwoven material.

Fig 5b illustrates an absorbent article 3 formed from the system according to the invention. The absorbent article is of belt type and comprises a chassis 10 and a belt 40. The belt 40 is attached to the chassis 10 adjacent to the back end 17. In fig 5b, the back end landing zone 22 on the chassis 10 is arranged on the outside of the chassis 10, on the back sheet 12. The back end landing zone 22 receives the center attachment means 41 on the belt 40. The back end landing zone 22 is adapted to integrate with the attachment technique of the center attachment means 41, such as glue, tape, loop-hook or the like. The length of the center attachment means 41 can be as long as the width of the back portion 18c or shorter. The center attachment means 41 may be provided in one or more portions along the belt 40. The one or more portions of the center attachment means 41 may extend in a length corresponding to the width of the back portion 18c of the chassis 10.

When the absorbent article 3 is being attached around the waist of a user, the left and right portions 43a, 43b of the belt 40 extends from the back of the user around the waist towards the front side. The front portion 18a of the chassis 10 faces the front of the user, and the left and right portions 43a, 43b of the belt 40 are attached to the outside of the chassis by means of the left and right attachment means 42a, 42b. The tape in the left and right attachment means 42a, 42b adhere to the back sheet 12 of the chassis 10. The belt type absorbent article 3 may be placed around the waist of a user in an alternative way by first attaching the left and right portions 43a, 43b of the belt 40 to each other to form a belt around the waist. One way of attaching the left and right portions 43a, 43b to each other is to attach the left and right attachment means 42a, 42b to each other. Another way of attaching the left and right portions 43a, 43b to each other is to place the two portions overlapping each other such that left attachment means 42a is attached to the inside of the right portion 43b, and the right attachment means 42b is attached to the outside of the left portion 43a. Alternatively, the right attachment means 42b is attached to the inside of the left portion 43a, and the left attachment means 42a is attached to the outside of the right portion 43b. The left and right attachment means 42a, 42b may then be provided on different sides of the belt 40, i.e. one of them is on the inside and the other one on the outside of the belt 40. Then the inside of the front portion 18a of the chassis 10 is attached to the belt 40 by attachment means provided on the landing zones 20a, 20b and/or 23. The landing zones 20a, 20b and 23 may all be adapted to adhere to the outside of the belt 40. Alternatively, at least one of the landing zones 20a, 20b, 23 is adapted to adhere to the outside of the belt 40. The landing zones 20a, 20b, 23 are provided on the inside of the chassis 10.

In an alternative embodiment, the belt 40 may be provided in two parts. The two parts of the belt are both attached to the back portion 18c of the chassis 10 such that one of the parts extends to the left and one to the right from the chassis. The two parts then together forms a belt similar to the belt 40 in fig 5a, 5b.

The belt 40 may be refastenable to a chassis 10 and thereby reusable. The belt 40 may also be washable.

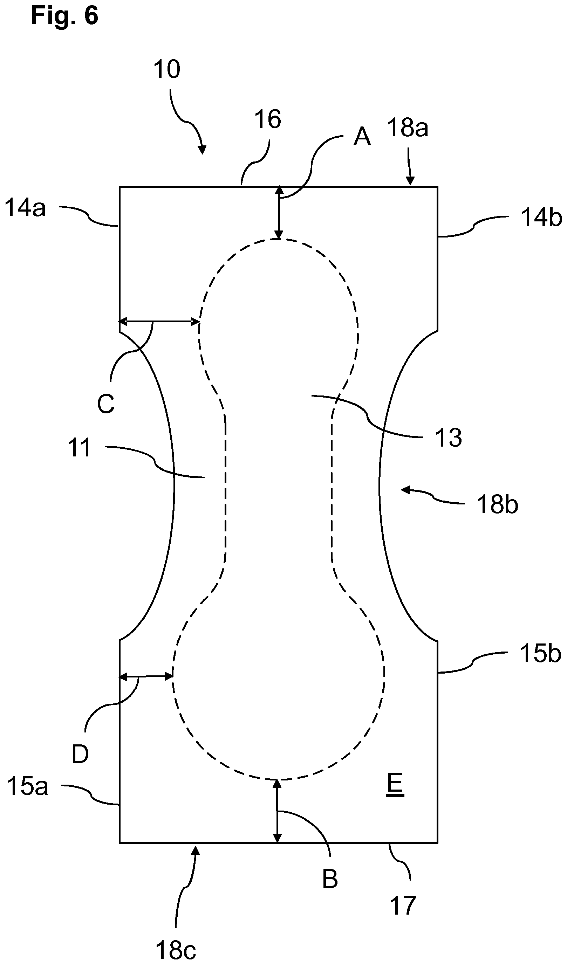

Fig 6 illustrates a chassis 10 according to the invention comprising a front portion 18a with a front end 16 and left and right front side edges 14a, 14b, a back portion 18c with a back end 17 and left and right back side edges 15a, 15b, and a crotch portion 18b connecting the front portion 18a with the back portion 18c. An absorbent core 13 is arranged between a liquid impermeable back sheet 12 and a liquid permeable top sheet 11. The back sheet 12 and the top sheet 11 extend over the complete extension area of the chassis 10. The absorbent core 13 has a smaller extension area than the complete chassis 10. In other words, there is a surplus extension area E of the chassis where the top sheet and the back sheet are in direct contact with each other, i.e. where there is no absorbent core arranged between the top sheet and the back sheet. The complete extension area of the chassis 10 is the sum of the extension area of the core and the surplus extension area E of the chassis. The complete extension area of the chassis 10 may be about 200% of the extension area of the absorbent core 13. In other words, the extension area of the absorbent core is about 50 % of the complete extensions area of the chassis, and the surplus extension area of the chassis is about 50 % of the complete extension area of the chassis. The absorbent core 13 is arranged in the chassis such that there is a distance A from its front end to the front end 16 of the chassis 10. The distance A is preferably more than 5 cm, preferably at least 6 cm, more preferably between 6 and 15 cm. According to an example, the distance A is 7 cm. The absorbent core 13 is further arranged in the chassis such that there is a distance B from its back end to the back end 17 of the chassis 10. The distance B is at least 5 cm, preferably at least 6 cm, more preferably between 6 and 15 cm. According to an example, the distance B is 7 cm. The absorbent core 13 is further arranged in the chassis such that there is a distance C from its left front side edge to the left front side edge 14a of the chassis 10. The distance C is at least 5 cm, preferably between 5 and 15 cm. According to an example, the distance C is 9 cm. The distance from the right front side edge of the absorbent core 13 to the right front side edge 14b of the chassis is about the same as the distance C from the left front side edge of the absorbent core to the left front side edge 14a of the chassis 10. The absorbent core 13 is further arranged in the chassis such that there is a distance D from its left back side edge to the left back side edge 15a of the chassis 10. The distance D is at least 5 cm, between 5 and 15 cm. According to an example, the distance D is 6 cm. The distance from the right back side edge of the absorbent core 13 to the right back side edge 15b of the chassis is about the same as the distance D from the left back side edge of the absorbent core to the left back side edge 15a of the chassis 10.

This disclosure also describes a pad (see e.g. figure 3 and figure 6). The absorbent article, e.g. the pad, has a chassis 10 having a front portion 18a and a back portion 18c and a longitudinal extension along a longitudinal axis X from a front end 16 of the front portion 18a towards a back end 17 of the back portion 18c. The chassis 10 further comprising a crotch portion 18b connecting the front portion 18a to the back portion 18c. The front portion has left and right front side edges 14a, 14b and the back portion has left and right back side edges 15a, 15b. The chassis further comprises a liquid permeable top sheet 11, and a liquid impermeable back sheet 12, wherein an absorbent core 13 is arranged between the top sheet 11 and the back sheet 12.

There is a distance A from a front end of the absorbent core to the front end 16 of the chassis 10. which distance A is more than 5 cm, preferably 6-25 cm, more preferably 6-15 cm, and most preferably 6.5 to 9 cm. According to a specific embodiment, the distance A may be 7 cm.

There is a distance B from a back end of the absorbent core to the back end 17 of the chassis 10 that is at least 5 cm, preferably 6-25 cm, more preferably 6-15 cm, and most preferably 6.5-9 cm. According to a specific embodiment, the distance B may be 7 cm.

There is a distance C from a front side edge of the absorbent core to a front side edge 14a, 14b of the chassis 10 that is at least 5 cm, preferably 5-25 cm, more preferably 5-15 cm, and most preferably 7-11 cm. According to a specific embodiment, the distance C may be 9 cm.

There is a distance D from a back side edge of the absorbent core to a back side edge 15a, 15b of the chassis 10 that is at least 5 cm, preferably 5-25 cm, more preferably 5-15 cm, and most preferably 5.5-9 cm. According to a specific embodiment, the distance D may be 6 cm.

By a pad according to at least some of the above mentioned characteristics there is provided a pad that can easily be fitted well into an undergarment according to the need of that specific user. Further, the part of the chassis where the back sheet and the top sheet are in direct contact with each other, also called the surplus extension area of the chassis, is relatively large, providing space for receiving add-on means such as wings etc. Thereby, it may be possible to convert the pad to an all-in one type article attachable around the waist of a user.

In the drawings and specification, there have been disclosed preferred embodiments and examples of the invention and, although specific terms are employed, they are used in a generic and descriptive sense only and not for the purpose of limitation, the scope of the invention being set forth in the following claims.

## Claims

1. System for forming an absorbent article of pad type or all-in-one type attachable around the waist of a user, the system comprising:
a chassis (10) having a front portion (18a) and a back portion (18c) and a longitudinal extension along a longitudinal axis (X) from a front end (16) of the front portion (18a) towards a back end (17) of the back portion (18c), the chassis (10) further comprising a crotch portion (18b) connecting the front portion (18a) to the back portion (18c), the front portion having left and right front side edges (14a, 14b), the back portion having left and right back side edges (15a, 15b), the chassis further comprising,
a liquid permeable top sheet (11), and
a liquid impermeable back sheet (12),
wherein an absorbent core (13) is arranged between the top sheet (11) and the back sheet (12),
**characterized in that**:
the system being arranged such that the chassis forms a pad type absorbent article,
the system further comprises at least one first add-on means (30, 32) adapted to be attached with a first end to the chassis (10) such that the first add-on means extend substantially transversal to the longitudinal axis of the chassis, to enable formation of an absorbent article of all-in-one type that is attachable around the waist of a user;
there is a distance (A) from a front end of the absorbent core to the front end (16) of the chassis (10) that is more than 5 cm;
there is a distance (B) from a back end of the absorbent core to the back end (17) of the chassis (10) that is at least 5 cm;
there is a distance (C) from a front side edge of the absorbent core to the front side edge (14a, 14b) of the chassis (10) that is at least 5 cm; and
there is a distance (D) from a back side edge the absorbent core to the back side edge (15a, 15b) of the chassis (10) that is at least 5 cm.

2. System according to claim 1, wherein the system further comprises at least one second add-on means (40) that differs from the first add-on means in size or shape, adapted to be attached to the chassis to form an absorbent article (3), wherein the system is arranged such that the chassis (10) is adapted to be used for forming absorbent articles (1,2, 3) both by combining the chassis with a first add-on means (30, 32) to form an all-in-one type absorbent article (1, 2) that is attachable around the waist of a user, by combining the chassis (10) with a second add-on means (40) to form another type of all-in-one type absorbent article (3) that is attachable around the waist of a user, or by using the chassis (10) as a pad type absorbent article.

3. System according to any of the preceding claims, wherein the first add-on means (30, 32) comprises at least one slip type wing and to which at least one wing the chassis (10) is adapted to be attached to form an absorbent article (1, 2) of slip type that is attachable around the waist of a user.

4. System according to claim 2, wherein the second add-on means (40) is a belt adapted to be attached around the waist of a user and to which belt the chassis (10) is adapted to be attached to form a belted-type absorbent article that is attachable around the waist of a user.

5. System according to any of the preceding claims, wherein the chassis (10) comprises at least one landing zone (20, 21, 22) for receiving a first add-on means (30, 32).

6. System according to any of the preceding claims, wherein the chassis (10) is provided with means for attachment to an undergarment.

7. System according to any of the preceding claims, wherein the system further comprises a pad attachment means, adapted to be attached to the chassis (10), and adapted to facilitate the attachment of a chassis used as a pad type absorbent article to an undergarment.

8. System according to any of the preceding claims, wherein the extension area of the chassis (10) is within the range of 150-300%, preferably 170-250% of the extension area of the absorbent core (13).

9. System according to any of the preceding claims, wherein the distance (A) from the front end of the absorbent core to the front end (16) of the chassis (10) is 6-25 cm, preferably 6-15 cm, and more preferably 6.5 to 9 cm.

10. System according to any of the preceding claims, wherein the distance (B) from the back end of the absorbent core to the back end (17) of the chassis (10) is 6-25 cm, preferably 6-15 cm, and more preferably 6.5-9 cm.

11. System according to any of the preceding claims, wherein the distance (C) from the front side edge of the absorbent core to the front side edge (14a, 14b) of the chassis (10) is 5-25 cm, preferably 5-15 cm, and more preferably 7-11 cm.

12. System according to any of the preceding claims, wherein the distance (D) from the back side edge the absorbent core to the back side edge (15a, 15b) of the chassis (10) is 5-25 cm, preferably 5-15 cm, and more preferably 5.5-9 cm.

## Patentansprüche

1. System zum Bilden eines absorptionsfähigen Artikels vom Blocktyp oder vom Komplett-Typ, der um die Taille eines Benutzers befestigt werden kann, wobei das System Folgendes umfasst:
einen Träger (10), der einen Vorderteil (18a) und einen Hinterteil (18c) und eine längliche Verlängerung entlang einer Längsachse (X) von einem Vorderende (16) des Vorderteils (18a) zu einem hinteren Ende (17) des Hinterteils (18c) hat, wobei der Träger (10) ferner einen Verzweigungsteil (18b) hat, der den Vorderteil (18a) mi dem Hinterteil (18c) verbindet, wobei der Vorderteil linke und rechte Vorderseitenkanten (14a, 14b) hat, wobei der Hinterteil linke und rechte Rückseitenkanten (15a, 15b) hat und der Träger ferner umfasst:
ein flüssigkeitsdurchlässiges oberes Sheet (11), und
ein flüssigkeitsdurchlässiges hinteres Sheet (12),
wobei ein absorptionsfähiger Kern (13) zwischen dem oberen Sheet (11) und dem hinteren Sheet (12) angeordnet ist,
**dadurch gekennzeichnet, dass**:
das System dafür ausgelegt ist, dass der Träger einen absorptionsfähigen Artikel vom Blocktyp bildet,
wobei das System ferner mindestens ein erstes Erweiterungsmittel (30, 32) umfasst, das dafür ausgelegt ist, mit einem ersten Ende am Träger (10) derart befestigt zu werden, dass das erste Erweiterungsmittel sich im Wesentlichen transversal zur Längsachse des Trägers erstreckt, um die Bildung eines absorptionsfähigen Artikels vom Kompletttyp zu ermöglichen, das um die Taille eines Benutzers gelegt und befestigt werden kann;
es besteht ein Abstand (A) von einem Vorderende des absorptionsfähigen Kerns bis zum Vorderende (16) des Trägers (10), der mindestens 5 cm beträgt;
es besteht ein Abstand (B) von einem Hinterende des absorptionsfähigen Kerns bis zum Hinterende (17) des Trägers (10), der mindestens 5 cm beträgt;
es besteht ein Abstand (C) von einer Vorderseitenkante des absorptionsfähigen Kerns bis zur Vorderseitenkante (14a, 14b) des Trägers (10), der mindestens 5 cm beträgt; und
es besteht ein Abstand (D) von einer Hinterseitenkante des absorptionsfähigen Kerns bis zur Hinterseitenkante (15a, 15b) des Trägers (10), der mindestens 5 cm beträgt.

2. System nach Anspruch 1, wobei das System ferner mindestens ein zweites Erweiterungsmittel (40) umfasst, das sich vom ersten Erweiterungsmittel in Größe oder Form unterscheidet, das dafür ausgelegt ist, am Träger befestigt zu werden, um einen absorptionsfähigen Artikel (3) zu bilden, wobei das System dafür ausgelegt ist, dass der Träger (10) zum Bilden von absorptionsfähigen Artikeln (1, 2, 3) verwendet werden kann, sowohl durch Kombinieren des Trägers mit einem ersten Erweiterungsmittel (30, 32), um einen absorptionsfähigen Artikel (1, 2) vom Kompletttyp zu bilden, der um die Taille eines Benutzers gelegt und befestigt werden kann, und durch Kombinieren des Trägers (10) mit einem zweiten Erweiterungsmittel (40), um eine andere Art von absorptionsfähigem Artikel (3) vom Kompletttyp zu bilden, der um die Taille eines Benutzers befestigbar gelegt werden kann, oder durch Verwenden des Trägers (10) als ein absorptionsfähiger Artikel vom Blocktyp.

3. System nach einem der vorherigen Ansprüche, wobei das erste Erweiterungsmittel (30, 32) mindestens einen Flügel vom Sliptyp umfasst und an dem mindestens ein Flügel der Träger (10) befestigt werden kann, um einen absorptionsfähigen Artikel (1, 2) vom Sliptyp zu bilden, der um die Taille eines Benutzers befestigbar gelegt werden kann.

4. System nach Anspruch 2, wobei das zweite Erweiterungsmittel (40) ein Gürtel ist, der um die Taille eines Benutzers befestigbar gelegt werden kann, und an dem Gürtel kann der Träger (10) befestigt werden, um einen absorptionsfähigen Artikel vom Gürteltyp zu bilden, der befestigbar um die Taille eines Benutzers gelegt werden kann.

5. System nach einem der vorherigen Ansprüche, wobei der Träger (10) mindestens eine Aufsetzzone (20, 21, 22) zum Aufnehmen eines ersten Erweiterungsmittels (30, 32) umfasst.

6. System nach einem der vorherigen Ansprüche, wobei der Träger (10) mit Mitteln zum Befestigen an Unterwäsche versehen ist.

7. System nach einem der vorherigen Ansprüche, wobei das System ferner ein Blockbefestigungsmittel umfasst, das zum Befestigen am Träger (10) und zum Erleichtern der Befestigung eines Chassis, das als absorptionsfähiger Artikel vom Blocktyp verwendet wird, an Unterwäsche ausgelegt ist.

8. System nach einem der vorherigen Ansprüche, wobei der Erweiterungsbereich des Trägers (10) innerhalb des Bereichs von 150 - 300 %, vorzugsweise 170 - 250 % des Erweiterungsbereichs des absorptionsfähigen Kerns (13) liegt.

9. System nach einem der vorherigen Ansprüche, wobei der Abstand (A) vom Vorderende des absorptionsfähigen Kerns bis zum Vorderende (16) des Trägers (10) 6 - 25 cm, vorzugsweise 6 - 15 cm und am besten 6,5 bis 9 cm beträgt.

10. System nach einem der vorherigen Ansprüche, wobei der Abstand (B) vom Hinterende des absorptionsfähigen Kerns bis zum Hinterende (17) des Trägers (10) 6 - 25 cm, vorzugsweise 6 - 15 cm und am besten 6,5 - 9 cm beträgt.

11. System nach einem der vorherigen Ansprüche, wobei der Abstand (C) von der Vorderseitenkante des absorptionsfähigen Kerns bis zur Vorderseitenkante (14a, 14b) des Trägers (10) 5 - 25 cm, vorzugsweise 5 - 15 cm und am besten 7 - 11 cm beträgt.

12. System nach einem der vorherigen Ansprüche, wobei der Abstand (D) von der Hinterseitenkante des absorptionsfähigen Kerns bis zur Hinterseitenkante (15a, 15b) des Trägers (10) 5 - 25 cm, vorzugsweise 5 - 15 cm und am besten 5,5 - 9 cm beträgt.

## Revendications

1. Système pour la formation d'un article absorbant de type coussin ou de type tout en un pouvant être fixé autour de la taille d'un utilisateur, ce système comprenant :
un châssis (10) doté d'une partie avant (18a) et d'une partie arrière (18c) et d'une extension longitudinale le long d'un axe longitudinal (X) depuis une extrémité avant (16) de la partie avant (18a) jusqu'à une extrémité arrière (17) de la partie arrière (18c), le châssis (10) comprenant en outre une partie entrejambe (18b) connectant la partie avant (18a) à la partie arrière (18c), la partie avant comportant des bords latéraux gauche et droit (14a, 14b), la partie arrière comportant des bords latéraux arrière gauche et droit (15a, 15b), le châssis comprenant en outre :
une feuille supérieure perméable au liquide (11) et
une feuille arrière imperméable au liquide (12),
un noyau absorbant (13) étant disposé entre la feuille supérieure (11) et la feuille arrière (12),
**caractérisé en ce que** :
le système est conçu de manière à ce que le châssis forme un article absorbant de type coussin,
le système comprend en outre au moins un premier moyen rapporté (30, 32) apte à être fixé par une première extrémité au châssis (10) de manière à ce que le premier moyen rapporté s'étende sensiblement transversalement à l'axe longitudinal du châssis pour permettre la formation d'un article absorbant de type tout en un qui peut être fixé autour de la taille d'un utilisateur ;
il y a une distance (A) depuis une extrémité avant du noyau absorbant jusqu'à l'extrémité avant (16) du châssis (10) qui est de plus de 5 cm ;
il y a une distance (B) depuis une extrémité arrière du noyau absorbant jusqu'à l'extrémité arrière (17) du châssis (10) qui est d'au moins 5 cm ;
il y a une distance (C) depuis un bord latéral avant du noyau absorbant jusqu'au bord latéral avant (14a, 14b) du châssis (10) qui est d'au moins 5 cm ; et
il y a une distance (D) depuis un bord latéral arrière du noyau absorbant jusqu'au bord latéral arrière (15a, 15b) du châssis (10) qui est d'au moins 5 cm.

2. Système selon la revendication 1, ce système comprenant en outre au moins un second moyen rapporté (40) qui diffère du premier moyen rapporté par la taille ou la forme et est apte à être fixé au châssis pour former un article absorbant (3), le système étant conçu de manière à ce que le châssis (10) soit apte à être utilisé pour former des articles absorbants (1, 2, 3) à la fois en combinant le châssis avec un premier moyen rapporté (30, 32) pour former un article absorbant de type tout en un (1, 2) qui peut être fixé autour de la taille d'un utilisateur, en combinant le châssis (10) avec un second moyen rapporté (40) pour former un autre type d'article absorbant tout en un (3) qui peut être fixé autour de la taille d'un utilisateur ou en utilisant le châssis (10) comme article absorbant de type coussin.

3. Système selon l'une quelconque des revendications précédentes, dans lequel le premier moyen rapporté (30, 32) comprend une aile en forme de slip à laquelle au moins une aile du châssis (10) est apte à être fixée pour former un article absorbant (1, 2) en forme de slip qui peut être fixé autour de la taille d'un utilisateur.

4. Système selon la revendication 2, dans lequel le second moyen rapporté (40) est une ceinture apte à être fixée autour de la taille d'un utilisateur et à laquelle ceinture le châssis (10) est apte à être fixé pour former un article absorbant de type ceinturé qui peut être fixé autour de la taille d'un utilisateur.

5. Système selon l'une quelconque des revendications précédentes, dans lequel le châssis (10) comprend au moins une zone de positionnement (20, 21, 22) pour recevoir un premier moyen rapporté (30, 32).

6. Système selon l'une quelconque des revendications précédentes, dans lequel le châssis (10) est pourvu d'un moyen de fixation à un sous-vêtement.

7. Système selon l'une quelconque des revendications précédentes, dans lequel le système comprend en outre un premier moyen de fixation de coussin, apte à être fixé au châssis (10) et apte à faciliter la fixation d'un châssis utilisé comme article absorbant de type coussin à un sous-vêtement.

8. Système selon l'une quelconque des revendications précédentes, dans lequel la zone d'extension du châssis (10) constitue de l'ordre de 250 - 300 %, de préférence 170 - 250 %, de la zone d'extension du noyau absorbant (13).

9. Système selon l'une quelconque des revendications précédentes, dans lequel la distance (A) depuis l'extrémité avant du noyau absorbant jusqu'à l'extrémité arrière (16) du châssis (10) est de 6 - 25 cm, de préférence 6 - 15 cm, et plus préférentiellement, de 6,5 à 9 cm.

10. Système selon l'une quelconque des revendications précédentes, dans lequel la distance (B) depuis l'extrémité arrière du noyau absorbant jusqu'à l'extrémité arrière (17) du châssis (10) est de 6 - 25 cm, de préférence 6 - 15 cm, et plus préférentiellement, de 6,5 - 9 cm.

11. Système selon l'une quelconque des revendications précédentes, dans lequel la distance (C) depuis le bord latéral avant du noyau absorbant jusqu'au bord latéral avant (14a, 14b) du châssis (10) est de 5 - 25 cm, de préférence 5 - 15 cm, et plus préférentiellement, de 7 - 11 cm.

12. Système selon l'une quelconque des revendications précédentes, dans lequel la distance (D) depuis le bord latéral arrière du noyau absorbant jusqu'au bord latéral arrière (15a, 15b) du châssis (10) est de 5 - 25 cm, de préférence 5 - 15 cm, et plus préférentiellement, de 5,5 - 9 cm.
